# EUROPEAN PATENT APPLICATION

(11) **EP 4 285 944 A1**
(43) Date of publication of application: **06.12.2023**
(21) Application number: 22176711.4
(22) Date of filing: 01.06.2022
(51) Int. Cl.: A61L 15/32, A61L 15/42, B05D 1/26, B05D 3/02

(54) **METHOD OF MAKING A COMPOSITION WITH A FILM-COATED POROUS MATERIAL**

(71) Applicant: MedSkin Solutions Dr. Suwelack AG, 20354 Hamburg (DE)
(72) Inventor: Ferro, Diana, 20249 Hamburg (DE); Völker, Annalena, 48301 Nottuln (DE); Wogram, Marco, 22113 Oststeinbek (DE)
(74) Representative: Kilger, Ute

(57) **Abstract**

The present invention relates to a method of making a composition comprising a film-coated porous material and a composition obtainable by this method. The invention further relates to the use of this product in cosmetic skin treatment and treatment of stagnating wounds, split skin graft and ulcers.

## Description

### Technical field

The present invention relates to a method of making a composition comprising a film-coated porous material and a composition obtainable by this method. The invention further relates to the use of this product in cosmetic skin treatment and treatment of stagnating wounds, split skin graft and ulcers.

### Background of the invention

A variety of coating techniques are employed in order to deposite a material onto a substrate, which include chemical or physical vapor deposition, electrochemical techniques, spraying, slot-die coating, etc. In particular, the use of electrostatic powder coating techniques to coat electrically conductive substrates, such as metals, is well known. By this method, a powder coating material is statically charged and then sprayed or blown onto a surface of a conductive material to which it adheres. The material is impregnated with the powder by means of electrostatic attraction between the positively charged or ionized powder and negatively charged surface of the conductive material or vise verca. This method is particularly used for painting metal articles.

Another well-established technique for the application of solutions onto typically planar substrates is slot-die coating. Slot-die coating allows micron-thick layers to be reliably coated on a flat substate surface at relatively high speeds, e.g. as indicated in US7097673B2. The coating material is typically dissolved or suspended into a solution or slurry and applied onto a surface of the substrate through a precise coating head known as a slot-die. Slot-die coating is thus a continuous coating technique which delivers quantitatively precise amounts of material, typically of low viscosity, onto a surface at a relatively high speed.

Common usages of slot-die coating technologies are generally limited to smooth nonporous materials, such as photographic films and papers. An important technical issue related to coating porous substrates is how to predict and control the fluid penetration into pores, which directly affects the appearance, properties, and performance of the resulting material. Porous materials are highly attractive as controlled drug carriers because of their large surface area, tunable pore size and mechanical stability. In particular, porous materials are widely used in medical devices as antibacterial, antithrombosis and wound healing agents. However, uncontrolled penetration of a liquid coating containing a healing agent into pores may negatively impact the healing properties of such a device. Therefore, the stability of the liquid coating onto a surface of a porous material is of particular importance for devices for topical administration.

### Description of the invention

In one aspect, the present invention relates to a method of making a composition comprising a porous material, wherein said porous material is essentially flat and comprises a plurality of open and interconnected pores with pore surfaces, comprising the steps of:
a) Providing a porous material, wherein said porous material is essentially flat and comprises a plurality of open and interconnected pores with pore surfaces, wherein the density of said porous material is in the range of from 0,01 to 1 g/cm³ and wherein said pores have an average diameter in the range of from 10 to 150 µm;
b) Providing a coating solution, wherein the viscosity of said coating solution is in the range of from 1 to 20 Pa·s;
c) Providing an applicator means for applying the coating solution onto the surface of said porous material, wherein said applicator means comprises a slot-die;
d) Applying a quantitative amount of the coating solution onto the surface of said porous material to coat said porous material and form a liquid layer by relatively moving said porous material and the applicator means with respect to each other at a speed in the range of from 0,1 to 10 m per minute;
e) Drying said liquid layer.

The method according to the present invention based on a slot-die technique allows to cover the surface of the porous material with a thin layer of a film coating, which predominantly remains on the surface of the porous material and does not penetrate inside the pores. This effect is achieved by balancing the density of the porous material and the viscosity of the applied coating solution. It was found that coating solutions with the viscosities in the range of from 1 to 20 Pa·s can be applied to porous materials with a very low density starting from 0,01 g/cm³. The density of the porous material increases by 10 to 30% after the coating, which contributes to maintaining the coating material on the surface of the substrate. At the same time, the porous structure of the porous material is maintained what is essential for controlled delivery of a healing agent.

In another aspect, the invention relates to a composition comprising a porous material obtainable by the method according to the invention.

In yet another aspect, the invention relates to use of a composition comprising a porous material obtainable by the method according to the invention in cosmetic skin treatment, such as treatment or prevention of wrinkles, skin irritation, and as a medicament, in particular for treatment of stagnating wounds, split skin graft and ulcers

### Detailed description

In one aspect, the present invention relates to a method of making a composition comprising a porous material, wherein said porous material is essentially flat and comprises a plurality of open and interconnected pores with pore surfaces, comprising the steps of:
a) Providing a porous material, wherein said porous material is essentially flat and comprises a plurality of open and interconnected pores with pore surfaces, wherein the density of said porous material is in the range of from 0,01 to 1 g/cm³, preferably in the range of from 0,02 to 0,05 g/cm³, most preferred in the range of from 0,02 to 0,04 g/cm³ and wherein said pores have an average diameter in the range of from 10 to 150 µm;
b) Providing a coating solution, wherein the viscosity of said coating solution is in the range of from 1 to 20 Pa·s, preferably from 8 to 15 Pa·s, more preferably from 10 to 14 Pa·s;
c) Providing an applicator means for applying the coating solution onto the surface of said porous material, wherein said applicator means comprises a slot-die;
d) Applying a quantitative amount of the coating solution onto the surface of said porous material to coat said porous material and form a liquid layer by relatively moving said porous material and the applicator means with respect to each other at a speed in the range of from 0,1 to 10 m per minute;
e) Drying said liquid layer.

In one embodiment, the density of said porous material increases by 10 to 30%, preferably by 10 to 20% after the coating, in the method of making a composition comprising a porous material.

In one embodiment, the quantitative amount of the coating solution applied onto the surface of said porous material in step d) is in the range of from 0,5 to 200 g/m², preferably in the range of from 10 to 100 g/m², most preferred in the range of from 10 to 30 g/m². In one embodiment, the quantitative amount of the coating solution applied onto the surface of said porous material in step d) is in the range of from 0,5 to 200 g/m². In one embodiment, the quantitative amount of the coating solution applied onto the surface of said porous material in step d) is in the range of from 10 to 100 g/m². In one embodiment, the quantitative amount of the coating solution applied onto the surface of said porous material in step d) is in the range of from 10 to 30 g/m².

In one embodiment the thickness of said porous material is in the range of from 100 to 5000 µm, preferably in the range of from 500 to 3000 µm, more preferably in the range of from 1000 to 2000 µm.

In one embodiment the thickness of the coating after step e) is in the range of from 1 to 300 µm, preferably in the range of from 2 to 50 µm, more preferably in the range of from 5 to 10 µm.

In one embodiment, the coating solution in the method of making a composition comprising a porous material comprises a solvent component and a film forming component. In one embodiment, the solvent component is selected from the group consisting of water and an alcohol, such as ethanol, and mixtures thereof. In one embodiment, the solvent component is water. In a particular embodiment, the content of water in the coating solution is in the range of from 0,5 to 50 wt%, preferably in the range of from 2 to 35 wt%, more preferably in the range of from 3 to 30 wt%. In one embodiment, the film forming component is selected from the group consisting of hyaluronic acid (HA), carboxymethylcellulose (CMS), polyacrylate and polyurethane or mixtures thereof. In one embodiment, the film forming component is a mixture of hyaluronic acid (HA) and carboxymethylcellulose (CMS) and the solvent component is water. In one embodiment the weight ratio of hyaluronic acid (HA) and carboxymethylcellulose (CMS) in water ranges from 1 : 3 to 3 : 1, preferably from 1 : 2 to 2 : 1, most preferred the weight ratio of hyaluronic acid (HA) and carboxymethylcellulose (CMS) in water is 1 : 1. In one embodiment the coating solution comprises 0,5 to 3 wt% of hyaluronic acid (HA), 0,5 to 3 wt% of carboxymethylcellulose (CMS) and 94-99 wt% water. In one embodiment the coating solution comprises ca 1 wt% of hyaluronic acid (HA), ca 1 wt% of carboxymethylcellulose (CMS) and ca 98 wt% water.

In one embodiment, the coating solution in the method of making a composition comprising a porous material comprises an emulgator. In one embodiment the emulgator is selected from the group consisting of polyglyceryl-10 laurate, glycerine, sucrose stearate, methyl glucose sequistearate, glyceryl stearate, cetearyl glucoside, hydrogenated palm glycerides, polyethyleneglycole and mixtures thereof.

In one embodiment, the coating solution in the method of making a composition comprising a porous material comprises a further ingredient selected from the group consisting of Vitamin A, Vitamin B, Vitamin C, Vitamin D, Vitamin K and 4-[(1E,3S)-3-ethenyl-3,7-dimethylocta-1,6-dienyl]phenol (Bakuchiol) or mixtures thereof. Preferably the further ingredient is Vitamin D.

In one embodiment, the porous material in the method of making the composition comprising is in the form of a sheet, dressing or a rolled sheet. A rolled sheet is an advantageous form for continuous operating mode of coating the porous material with the coating solution.

The porous material in the method of making the composition is essentially flat, i.e. the thickness of the porous material throughout the length and the width of the material does not deviate from the average thickness of the porous material by more than ±20%, preferably ±10%. For example, with an average thickness of 1 mm the thickness of the material throughout its length and width remains in the range from 0,8 to 1,2 mm, preferably in the range of from 0,9 to 1,1 mm. With an average thickness of 2 mm the thickness of the essentially flat material throughout its length and width remains in the range of from 1,6 to 2,4 mm, preferably in the range of from 1,8 to 2,2 mm.

In one embodiment, the porous material in the method of making the composition comprising a porous material comprises at least 90 % by weight biomaterial, preferably the porous material comprises at least 95 % by weight biomaterial, more preferably the porous material comprises at least 98 % by weight of biomaterial, most preferred the porous material comprises at least 99% by weight biomaterial.

In one embodiment the density of the porous material in the method of making the composition comprising a porous material is in the range of from 0,01 to 1 g/cm³, preferably is in the range of from 0,02 to 0,05 g/cm³, more preferably is in the range of from 0,02 to 0,04 g/cm³, most preferred of from of 0,022 to 0,03 g/cm³.

In one embodiment, the pores in the porous material in the method of making the composition comprising a porous material have an average diameter in the range of from 10 to 150 µm.In one embodiment, the pores in the porous material in the method of making the composition comprising a porous material have an average diameter in the range of from 15 to 65 µm.In one embodiment, the pores in the porous material in the method of making the composition comprising a porous material have an average diameter in the range of from 15 to 40 µm.In one embodiment, the pores in the porous material in the method of making the composition comprising a porous material have an average diameter in the range of from 25 to 100 µm.

In one embodiment, the viscosity of the coating solution in the method of making the composition comprising a porous material is in the range of from 1 to 20 Pa·s. In a preferred embodiment, the viscosity of the coating solution in the method of making the composition comprising a porous material is in the range of from 8 to 15 Pa·s. In a more preferred embodiment, the viscosity of the coating solution in the method of making the composition comprising a porous material is in the range of from 10 to 14 Pa·s.

In one embodiment the porous material in the method of making the composition comprising a porous material has a density in the range of from 0,01 to 1 g/cm³ and the viscosity of the coating solution is in the range of from 1 to 20 Pa·s. In a preferred embodiment the porous material in the method of making the composition has a density in the range of from 0,02 to 0,05 g/cm³ and the viscosity of the coating solution is in the range of from 8 to 15 Pa·s. In a more preferred embodiment the porous material in the method of making the composition has a density in the range of from 0,02 to 0,04 g/cm³ and the viscosity of the coating solution is in the range of from 10 to 14 Pa·s.

In one embodiment, the porous material in the method of making the composition comprising a porous material is a biomaterial.

In one embodiment, the porous material in the method of making the composition comprising a porous material is selected from the group comprising natural and/or synthetic polymers or mixtures thereof, in particular polysaccharides, glucosaminoglycans, proteins or mixtures thereof.

In one embodiment, the porous material in the method of making the composition comprising a porous material is selected from the group consisting of collagen, alginate, e.g. calcium alginate, hyaluronic acid, cellulose and plant-based proteins, e.g. pie and soy, or a mixture thereof.

In one embodiment, the porous material in the method of making the composition comprising a porous material is collagen. In one embodiment, the porous material in the method of making the composition comprising a porous material is alginate, in particular calcium alginate.

In one embodiment the porous material in the method of making the composition comprising a porous material is a mixture of collagen and calcium alginate. In one embodiment, the porous material comprises collagen by weight in the range of from 80 to 98% and calcium alginate by weight in the range of from 2 to 20%. In a preferred embodiment, the porous material comprises collagen by weight in the range of from 85 to 95% and calcium alginate by weight in the range of from 5 to 15%. In particular, the porous material comprises collagen about 90% by weight and calcium alginate about 10% by weight.

In one embodiment, the collagene in the porous material in the method of making the composition comprising a porous material is animal derived native collagen with a triple helical structure.

In one embodiment the collagen in the porous material in the method of making the composition comprising a porous material is selected from the group comprising 1 type collagen, 3 type collagen, 5 type collagen or a mixture thereof.

In one embodiment of the method of making the composition comprising a porous material the application of the coating solution onto the surface of said porous material is conducted in the way that said porous material is positioned below the fixed slot-die and moves horizontally underneath said slot-die. In one embodiment the speed of moving said porous material is in the range of from 0,1 to 10 m per minute.

In one embodiment of the method of making the composition comprising a porous material the distance between the slot-die and the surface of the porous material is in a range of from 50 to 1000 µm, preferably from 100 to 800 µm, more preferably from 200 to 600 µm.

In one embodiment of the method of making the composition comprising a porous material the solvent component of the coating solution is water and the drying in step e) is conducted in the way that the final content of the water in the composition after drying is in the range of from 5 to 25 wt%, preferably in the range of from 10 to 18 wt%.

In one embodiment of the method of making the composition comprising a porous material the drying in step e) is conducted by supplying hot air to said liquid layer, wherein the temperature of the hot air is in the range from 30 to 100 °C, preferably in the range of from 35 to 50 °C.

In another aspect, the invention relates to a composition comprising a porous material obtainable by a method according to any of the preceding embodiments.

In third aspect, the invention relates to the composition comprising a porous material obtainable by a method according to any of the preceding embodiments for use as a medicament. In one embodiment, the invention relates to the composition comprising a porous material obtainable by a method according to any of the preceding embodiments for use in the treatment of stagnating wounds, split skin graft and ulcers.

In forth aspect, the invention relates to the composition comprising a porous material obtainable by a method according to any of the preceding embodiments for in cosmetic skin treatment. In one embodiment, the invention relates to the composition comprising a porous material obtainable by a method according to any of the preceding embodiments for use in treatment or prevention of wrinkles and skin irritation.

In fifth aspect, the invention relates to a use of the composition comprising a porous material obtainable by a method according to any of the preceding embodiments in the treatment of stagnating wounds, split skin graft and ulcers.

In sixth aspect, the invention relates to a use of the composition comprising a porous material obtainable by a method according to any of the preceding embodiments in cosmetic skin treatment, such as treatment or prevention of wrinkles and skin irritation.

In seventh aspect, the invention relates to the method of treatment of stagnating wounds, split skin graft and ulcers comprising administering the composition comprising a porous material obtainable by a method according to any of the preceding embodiments to a subject in the need thereof.

With the above context, the following consecutively numbered embodiments provide further specific aspects of the invention:
1. A method of making a composition comprising a porous material, wherein said porous material is essentially flat and comprises a plurality of open and interconnected pores with pore surfaces, comprising the steps of:
   a) Providing a porous material, wherein said porous material is essentially flat and comprises a plurality of open and interconnected pores with pore surfaces, wherein the density of said porous material is in the range of from 0,01 to 1 g/cm³, preferably in the range of from 0,02 to 0,05 g/cm³, most preferred in the range of from 0,02 to 0,04 g/cm³ and wherein said pores have an average diameter in the range of from 10 to 150 µm;
   b) Providing a coating solution, wherein the viscosity of said coating solution is in the range of from 1 to 20 Pa·s, preferably from 8 to 15 Pa·s, more preferably from 10 to 14 Pa·s;
   c) Providing an applicator means for applying the coating solution onto the surface of said porous material, wherein said applicator means comprises a slot-die;
   d) Applying a quantitative amount of the coating solution onto the surface of said porous material to coat said porous material and form a liquid layer by relatively moving said porous material and the applicator means with respect to each other at a speed in the range of from 0,1 to 10 m per minute;
   e) Drying said liquid layer.
2. The method of making a composition comprising a porous material according to embodiment 1, wherein the density of said porous material increases by 10 to 30%, preferably by 10 to 20% after the coating.
3. The method of making a composition comprising a porous material according to embodiment 1 or 2, wherein the quantitative amount of the coating solution applied onto the surface of said porous material in step d) is in the range of from 0,5 to 200 g/m², preferably in the range of from 10 to 100 g/m², most preferred in the range of from 10 to 30 g/m².
4. The method of making a composition comprising a porous material according to any of embodiments 1 to 3, wherein said coating solution comprises a solvent component and a film forming component.
5. The method of making a composition comprising a porous material according to embodiment 4, wherein said solvent component is water.
6. The method of making a composition comprising a porous material according to embodiment 5, wherein the content of water in said coating solution is in the range of from 0,5 to 50 wt%, preferably in the range of from 2 to 35 wt%, more preferably in the range of from 3 to 30 wt%.
7. The method of making a composition comprising a porous material according to any of embodiments 4 to 6, wherein said film forming component is selected from the group consisting of hyaluronic acid (HA), carboxymethylcellulose (CMS), polyacrylate, and polyurethane or mixtures thereof.
8. The method of making a composition comprising a porous material according to any of embodiments 1 to 7, wherein said coating solution comprises an emulsifier.
9. The method of making a composition comprising a porous material according to embodiment 8, wherein said emulsifier is selected from the group consisting of polyglyceryl-10 laurate, glycerine, sucrose stearate, methyl glucose sequistearate, glyceryl stearate, cetearyl glucoside, hydrogenated palm glycerides, polyethyleneglycole or mixtures thereof.
10. The method of making a composition comprising a porous material according to any of embodiments 1 to 9, wherein said coating solution comprises a further ingredient selected from the group consisting of Vitamin A, Vitamin B, Vitamin C, Vitamin K, and 4-[(1E,3S)-3-ethenyl-3,7-dimethylocta-1,6-dienyl]phenol (Bakuchiol), or mixtures thereof.
11. The method of making a composition comprising a porous material according to any of embodiments 1 to 10, wherein said porous material is a biomaterial.
12. The method of making a composition comprising a porous material according to embodiment 11, wherein said biomaterial is selected from the group comprising natural and/or synthetic polymers or mixtures thereof, in particular polysaccharides, glucosaminoglycans, proteins and/or synthetic polymers or mixtures thereof.
13. The method of making a composition comprising a porous material according to embodiment 11 or 12, wherein said biomaterial is selected from the group consisting of collagen, alginate, hyaluronic acid, cellulose, and plant-based proteins such as pie and soy.
14. The method of making a composition comprising a porous material according to embodiment 13, wherein said biomaterial is collagen.
15. The method of making a composition comprising a porous material according to embodiment 14, wherein said biomaterial is animal derived native collagen with a triple helical structure.
16. The method of making a composition comprising a porous material according to any of embodiments 1 to 15, wherein the application of the coating solution onto the surface of said porous material is conducted in the way that said porous material is positioned below the fixed slot-die and moves horizontally underneath said slot-die.
17. The method of making a composition comprising a porous material according to any of embodiments 1 to 16, wherein the distance between the slot-die and the surface of the porous material is in a range of from 50 to 1000 µm, preferably from 100 to 800 µm, more preferably 200 to 600 µm.
18. The method of making a composition comprising a porous material according to any of embodiments 1 to 17, wherein the drying in step e) is conducted by supplying hot air to said liquid layer, wherein the temperature of the hot air is in the range from 30 to 100 °C, preferably in the range of from 35 to 50 °C.
19. The method of making a composition comprising a porous material according to any of embodiments 1 to 18, wherein said porous material is in the form of a sheet, a dressing, or a rolled sheet.
20. A composition comprising a porous material obtainable by a method according to any of embodiments 1 to 19.
21. The composition comprising a porous material according to embodiment 20 for use as a medicament.
22. The composition comprising a porous material according to embodiment 20 for use in the treatment of stagnating wounds, split skin graft and ulcers.
23. The composition comprising a porous material according to embodiment 20 for use in cosmetic skin treatment, such as treatment or prevention of wrinkles and skin irritation.
24. Use of the composition comprising a porous material according to embodiment 20 in the treatment of stagnating wounds, split skin graft and ulcers.
25. Use of the composition comprising a porous material according to embodiment 20 in cosmetic skin treatment, such as treatment or prevention of wrinkles and skin irritation.
26. Method of treatment of stagnating wounds, split skin graft and ulcers comprising administering the composition according to embodiment 19 to a subject in the need thereof.

### Definitions

Listed below are definitions of various terms used to describe this invention. These definitions apply to the terms as they are used throughout this specification and claims unless otherwise limited in specific instances either individually or as part of a larger group. Unless defined otherwise all technical and scientific terms used herein generally have the same meaning as commonly understood by one of ordinary skill in the art to which this invention belongs.

As used herein, the articles "a" and "an" refer to one or to more than one (i.e. to at least one) of the grammatical object of the article. By way of example, "an element" means at least one element, i.e. an element or more than one element.

As used herein, the term "porous material" refers to a material comprising pores, i.e. cavities, channels or interstices, wherein the depth of the pores exceeds their average diameter.

Unless specifically defined, the term "thickness" related to a porous material refers to an average thickness of the porous material.

As used herein, the term "essentially flat" refers to the material with the thickness throughout the length and the width not deviating from the average thickness by more than ±20%, preferably ±10%.

As used herein, the term "sheet" refers to an essentially flat material, wherein the thickness of the material is in the range of from 1 to 8 mm.

As used herein, the terms "rolled material" and "rolled sheet" are used interchangeably and refer to the sheet of porous material wound cylindrically about a center axis of a roll with the possibility of removal of the material from the center or inner periphery of the roll.

As used herein, the term "dressing" refers to the composition comprising a film-coated porous material, which is further coated with an additional layer of a polymer, wherein said composition is in a form of a sheet.

As used herein the term "applicator means" refers to the means suitable for applying a liquid onto a surface of a material.

As used herein, the term "solvent component" refers to a liquid comprised of a single solvent or a mixture of solvents, which is suitable for solubilizing or dispersing one or more of a variety of substances. Non-limiting examples of such solvents include water and alcohols, such as ethanol or isopropanol.

As used herein, the term "film forming component" refers to a substance capable of forming a film upon application to a solid surface. In particular, the film forming component is applied to a surface in a form of a liquid layer and film is formed after the liquid layer dries in air. The non-limiting examples of film forming components include hyaluronic acid (HA), carboxymethyl cellulose (CMC), polyacrylate and polyurethane.

As used herein, the term "emulsifier" refers to a substance, which facilitates mixing of immiscible liquids with different polarities, e.g. water with lipophilic ingredients. Non-limiting examples of emulsifiers include polyglyceryl-10 laurate, polyglyceryl-10 laurate, glycerine, sucrose stearate, methyl glucose sequistearate, glyceryl stearate, cetearyl glucoside, hydrogenated palm glycerides, polyethyleneglycole.

As used herein, the term "coating" refers to a thin deposit of a material that substantially covers the surface of a substrate.

As used herein, the term "salt" refers to ionic a chemical compound consisting of an ionic assembly of a positively charged cation and a negatively charged anion.

As used herein, the term "biomaterial" refers to a natural or synthetic biocompatible material which is suitable for using in a medical device, intended to interact with biological systems. Non-limiting examples include collagen, gelatine, alginate and polysaccharides such as glycosaminoglycans.

As used herein, the term "polysaccharide" refers to polymers comprising a backbone comprised mainly of (at least 90%) monosaccharide repeating units and/or derivatized monosaccharide repeating units.

As used herein, the term "protein" or "polypeptide" refers to a polymer of two or more of the natural amino acids or non-natural amino acids.

As used herein the term "glycosaminoglycan" refers to a group of acid polysaccharides, each having a repeating unit of disaccharide consisting of an amino sugar and uronic acid or galactose.

As used herein, the term "alginate" refers to the anion of alginic acid. Therefore, the terms "alginate" and "alginate salt" are used interchangeable in the context of the present invention. The alginate salt can be, for example, calcium alginate. Alginate is a linear polymer formed by anions of β-D-mannuronic acid (M, β-D-mannuronate) and of α-L-guluronic acid (G, α-L-guluronate) bound by means of 1-4 glycosidic bonds.

As used herein, the term "collagen" refers to the extracellular family of fibrous proteins that are characterised by their stiff, triple-stranded helical structure. Three collagen polypeptide chains ("α-chains") are wound around each other to form this helical molecule.

As used herein the term "subject" refers to a human or a non-human mammal. Preferably the subject is human.

### Description of figures

Figure 1 shows a schematic view of a slot-die coating process.
Figure 2 shows a SEM (scanning electron microscope) image of a collagen matrix without coating, top view.
Figure 3 shows a SEM (scanning electron microscope) image of a collagen matrix with CMC coating, top view.

### Examples

### Example 1.

### Coating solution 1

- CMC (Carboxymethylcellulose): 3,3 wt%
- Water: 96,7 wt%.

### Coating solution 2

- Vitamin C: 25 wt%,
- CMC (Carboxymethylcellulose): 3,3 wt%,
- Water: 71,7 wt%

### Coating solution 3

- 4-[(1E,3S)-3-ethenyl-3,7-dimethylocta-1,6-dienyl]phenol (Bakuchiol): 5 wt %
- polyglyceryl-10 laurate: 5 wt%,
- CMC (Carboxymethylcellulose): 4 wt%,
- Water: 86 wt%

### Coating procedure:

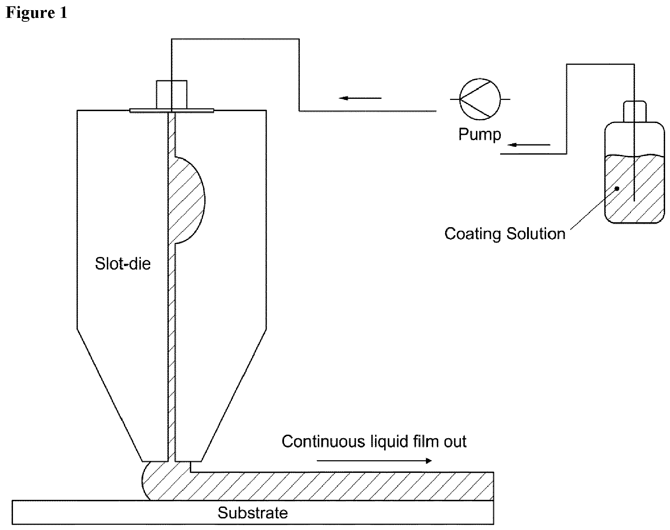

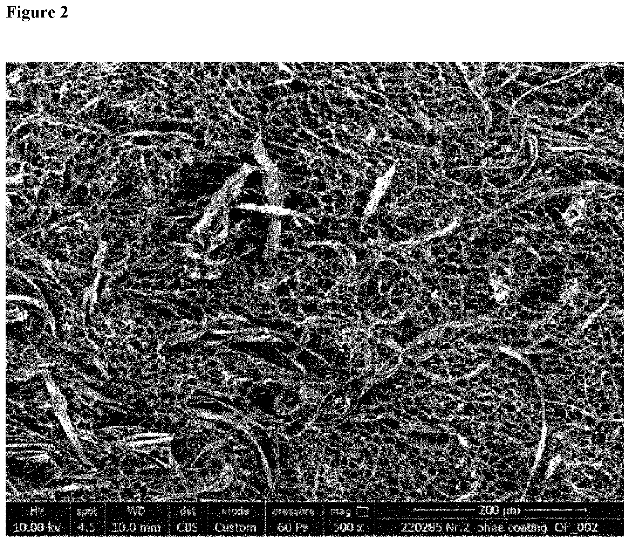

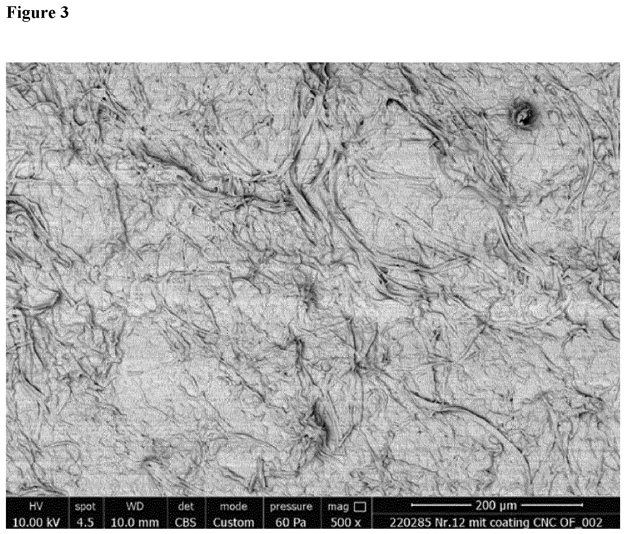

The coating was applied by means of a slot-die process. A schematic view of the process is depicted in Figure 1. The sequence of the process steps is as follows:
- A porous collagen matrix substrate with the dimensions of 46x33x0,15cm and the density of 0,022 g/cm² is placed on a movable table
- A slot-die is positioned at a distance of 700 µm from the plane of the substrate
- The table is arranged to move horizontally underneath the slot-die with the speed of 1 m/min
- When the slot-die reaches the position above the substrate, the pump is arranged to pump the coating solution with the flow rate 5 ml/min
- After the coating is evenly distributed across the substrate, the pump is switched off
- The applied coating is dried in an oven at 90°C for 2 minutes so that a thin coating film on the substrate is formed. The SEM images of the uncoated collagen matrix and the collagen matrix coated with CMC (Coating solution 1) are provided in Figures 2 and 3, respectively.

## Claims

1. A method of making a composition comprising a porous material, wherein said porous material is essentially flat and comprises a plurality of open and interconnected pores with pore surfaces, comprising the steps of:
a) Providing a porous material, wherein said porous material is essentially flat and comprises a plurality of open and interconnected pores with pore surfaces, wherein the density of said porous material is in the range of from 0,01 to 1 g/cm³, preferably in the range of from 0,02 to 0,05 g/cm³, most preferred in the range of from 0,02 to 0,04 g/cm³ and wherein said pores have an average diameter in the range of from 10 to 150 µm;
b) Providing a coating solution, wherein the viscosity of said coating solution is in the range of from 1 to 20 Pa·s, preferably from 8 to 15 Pa·s, more preferably from 10 to 14 Pa·s;
c) Providing an applicator means for applying the coating solution onto the surface of said porous material, wherein said applicator means comprises a slot-die;
d) Applying a quantitative amount of the coating solution onto the surface of said porous material to coat said porous material and form a liquid layer by relatively moving said porous material and the applicator means with respect to each other at a speed in the range of from 0,1 to 10 m per minute;
e) Drying said liquid layer.

2. The method of making a composition comprising a porous material according to claim 1, wherein the density of said porous material increases by 10 to 30%, preferably by 10 to 20% after the coating.

3. The method of making a composition comprising a porous material according to claim 1 or 2, wherein the quantitative amount of the coating solution applied onto the surface of said porous material in step d) is in the range of from 0,5 to 200 g/m², preferably in the range of from 10 to 100 g/m², most preferred in the range of from 10 to 30 g/m².

4. The method of making a composition comprising a porous material according to any of claims 1 to 3, wherein said coating solution comprises a solvent component and a film forming component.

5. The method of making a composition comprising a porous material according to claim 4, wherein said solvent component is water.

6. The method of making a composition comprising a porous material according to claim 5, wherein the content of water in said coating solution is in the range of from 0,5 to 50 wt%, preferably in the range of from 2 to 35 wt%, more preferably in the range of from 3 to 30 wt%.

7. The method of making a composition comprising a porous material according to any of claims 4 to 6, wherein said film forming component is selected from the group consisting of hyaluronic acid (HA), carboxymethylcellulose (CMS), polyacrylate, and polyurethane or mixtures thereof.

8. The method of making a composition comprising a porous material according to any of claims 1 to 7, wherein said coating solution comprises an emulsifier.

9. The method of making a composition comprising a porous material according to any of claims 1 to 8, wherein said porous material is a biomaterial.

10. The method of making a composition comprising a porous material according to claim 9, wherein said biomaterial is selected from the group comprising natural and/or synthetic polymers or mixtures thereof, in particular polysaccharides, glucosaminoglycans, proteins and/or synthetic polymers or mixtures thereof.

11. The method of making a composition comprising a porous material according to claim 10, wherein said biomaterial is collagen.

12. The method of making a composition comprising a porous material according to claim 11, wherein said biomaterial is animal derived native collagen with a triple helical structure.

13. A composition comprising a porous material obtainable by a method according to any of claims 1 to 12.

14. The composition comprising a porous material according to claim 13 for use as a medicament.

15. The composition comprising a porous material according to claim 13 for use in the treatment of stagnating wounds, split skin graft and ulcers.
